# EUROPEAN PATENT APPLICATION

(11) **EP 4 629 186 A1**
(43) Date of publication of application: **08.10.2025**
(21) Application number: 24168524.7
(22) Date of filing: 04.04.2024
(51) Int. Cl.: G06V 10/62, G06V 10/82, G06V 10/764

(54) **A METHOD AND SYSTEM FOR ANALYZING A METABOLITE PROFILE OF A SUBJECT**

(71) Applicant: HORAIZON technology B.V., 2645 LT Delfgauw (NL)
(72) Inventor: LEVIN, Evgeni, 2645 LT Delfgauw (NL)
(74) Representative: V.O.

(57) **Abstract**

A method and system for analyzing and/or estimating a metabolite profile of a subject. A digital image of a sample of feces of the subject is received by one or more processors. The digital image and/or one or more features extracted from the digital image is provided as input to a trained machine learning model which is configured to output a classification based on said input digital image and/or one or more features extracted from the digital image. Data indicative of one or more properties of the metabolome of the subject based on the output image classification is determined by the one or more processors.

## Description

### FIELD OF THE DISCLOSURE

The disclosure relates to a method of analyzing a metabolite profile of a subject. The disclosure also relates to a system configured for analyzing a metabolite profile of a subject. Furthermore, the disclosure relates to a method of determining an indication of a subject's health. Additionally, the disclosure relates to a method of training a machine learning model for analyzing a metabolite profile of a subject. Also, the disclosure relates to a computer program product.

### BACKGROUND TO THE DISCLOSURE

The significance of metabolites, primarily those resulting from the gut microbiome, in influencing a subject's health is well known. The metabolome, which encompasses the entire set of metabolites present within an organism, is closely connected to various aspects of an subject's health and performance, including but not limited to physical vitality, mental acuity, nutritional absorption, and immunological responsiveness. Fluctuations in the metabolome can serve as indicators for conditions such as but not limited to diabetes, where altered glucose levels are a primary metabolite of concern, or cardiovascular diseases, where lipid profiles offer critical insights. In neuropsychiatric domains, variations in neurotransmitter metabolites can reflect mental health states, influencing mood and cognitive functions. Thus, the metabolome acts as a dynamic interface, reflecting the intricate interplay between dietary intake, environmental exposures, genetic predispositions, and disease states, ultimately influencing an subject's overall physical and mental well-being. This holds true not only for human but also for animal nutrition and healthcare domains. The constitution and heterogeneity of metabolites within a subject are influenced by an various dietary habits and lifestyle decisions, compounded by both endogenous variables, including stress, nutritional deficits, or pathologies, thereby illustrating the interconnection between metabolite profiles and the subject's overall health status. This underscores the importance of sustaining a dynamic equilibrium in metabolite synthesis within the gastrointestinal tract. Specifically, dietary selections, with an emphasis on those that modulate microbiome, which in turn significantly affects metabolite production and composition, exert a significant impact on this homeostatic balance. In contrast, adverse lifestyle choices may precipitate a disruption in this metabolic equilibrium, leading to negative health outcomes, including but not limited to, cardiometabolic disorders, neurological disorders, cancers, and many other adverse health conditions.

The production and diversity of the metabolites in a subject is affected by dietary patterns and lifestyle choices, as well as external and internal factors such as stress, dietary imbalances, or illnesses, which shows the correlation of metabolites and overall health in a subject and the importance of maintaining a dynamic balance of metabolite production within the gut. Dietary choices, particularly those that influence microbial diversity such as fiber-rich and probiotic foods, can profoundly affect this balance. Conversely, negative lifestyle choices might lead to an imbalance in metabolite production, contributing to conditions like decreased overall health status, obesity or metabolic syndrome. As a substantial portion of human metabolites are microbially derived, therapeutic dietary strategies may be employed in order to manipulate microbial composition, and stability and to obtain and/or maintain a desired metabolite profile.

Unfortunately, and despite advancements in metabolomics analysis techniques (including but not limited to Mass Spectrometry, NMR) or any sequencing techniques), there is a gap in efficiently analyzing the metabolite profile in subject in a non-invasive, routine manner.

There is a strong need for a fast and efficient process for effectively analyzing and estimating a metabolite profile of a subject.

### SUMMARY

It is an object of the present disclosure to provide a method and system that alleviates least one of the previously delineated shortcomings.

Further, it is an object of this disclosure to enhance the analysis and characterization of the metabolite profile of a subject. This includes the detailed characterization and identification of the diverse metabolites present in the subject's feces.

Moreover, the metabolite profile encompasses the quantification of various metabolites, facilitating relative quantitative assessments among different metabolites.

Additionally or alternatively, the disclosure aims to augment the efficacy of metabolite analysis processes. Furthermore, an objective of this disclosure is to introduce a cost-efficient method and system for the assessment (e.g., estimation) of the metabolite profile in subjects, thereby facilitating a comprehensive and economical evaluation of metabolic activity. It is an object of the disclosure to provide for a method and a system that obviates at least one of the above-mentioned drawbacks.

Additionally or alternatively, it is an object of the disclosure to improve the analysis and/or characterization of a metabolite profile of a subject. The metabolite profile of a subject may include a characterization and/or identification of the various metabolites in the feces of the subject. The metabolite profile of a subject may also comprise a quantification of one or more of the various metabolites in the feces of the subject, including relative comparisons in quantity between one or more metabolites versus one or more other metabolites.

Additionally or alternatively, it is an object of the disclosure to improve the efficiency of metabolite analysis.

Additionally or alternatively, it is an object of the disclosure to provide for a cost-effective method and system for analyzing (e.g. estimating) the metabolite profile of a subject.

Thereto, the disclosure provides for a method for analyzing (e.g. estimating) a metabolite profile of a subject, comprising: receiving, by one or more processors, a digital image of a sample of feces of the subject. The subject can be human or animal. The method includes providing the digital image and/or one or more features extracted from the digital image as input to a trained machine learning model which is configured to output a classification based on said input digital image and/or one or more features extracted from the digital image; and determining, by the one or more processors, data indicative of one or more properties of the metabolite profile of the subject based on the output image classification.

Images of fecal samples are used for rapid and efficient analysis of the metabolome. The analysis can for example be used for rapid metabolite profiling, only using an image of the fecal sample or features extracted from said image. Advantageously, laboratory analysis is not needed. The process becomes less cumbersome and allows more easy, efficient and cost-effective monitoring of the metabolite profile of the subject. Additionally, this allows to perform multiple tests during a period of time, which would be unpractical if laboratory tests were to be conducted. For example, an image of a fecal sample may be monitored every day in order to track changes in the metabolite profile of the subject. As a result, it becomes significantly more easy to provide for personalized diet/ or execute any other health related intervention (e.g. drug administration). It can be determined whether the diet of the subject has to be adjusted in order to improve the metabolite balance (cf. prevent microbial imbalance). Thus, advantageously, a desired metabolite profile can be better obtained or maintained in this way.

Preferably, the digital image is a macroscopic image. The macroscopic image can e.g. have a field of view of at least 30 mm wide and at least 30 mm tall. The digital image can e.g. have a field of view of at least 75 mm wide and at least 75 mm tall, such as at least 150 mm wide and at least 150 mm tall. The digital image can advantageously be obtained using a camera of a mobile device, such as a mobile phone. Optionally, the digital image is an image of a fecal streak on a substrate.

Utilizing a fecal streak on a substrate can significantly improve the performance of the trained machine learning model. Furthermore, the machine learning model can be trained using less data. The training process can thus become more efficient if training data with fecal streaks are used for training the machine learning model (e.g. deep learning network model). This is an important benefit, as obtaining the training data may require expensive (laboratory) tests, for instance involving mass spectrometry, similar technologies or DNA/RNA sequencing.

Furthermore, as the accuracy of the trained machine learning model can be enhanced by using an image of a fecal streak on a substrate, it is possible to better obtain or maintain a desired metabolite profile of the subject. The trained machine learning model can provide improved predictability, whilst an efficient and simple method can be used. It is rather easy to make an image of a fecal streak on a substrate. In some cases, this image may be provided by a non-professional, for instance the subject may take the image of the fecal streak on a substrate.

Optionally, the fecal streak is obtained by smearing a layer of feces on the substrate.

By smearing a sample of feces on the substrate, a relatively thin layer of feces can be obtained. Such a thin layer of feces, which allow trained machine learning model to extract various features of the image such as but not limited to composition, color, texture, granularity and many others, may increase the performance of the trained machine learning model, and also improve training of the machine learning model.

Optionally, the thickness of the fecal streak layer is smaller than 3000 micrometer, more preferably smaller than 1000 micrometer, even more preferably smaller than 500 micrometer.

Optionally, the layer of feces is smeared on the substrate by means of a blade (or a brush). Using a blade, a relatively thin layer of feces can be applied on the substrate rather easily. In this way, a more uniform distribution of the feces over the substrate can be obtained. In this way, the grittiness of the feces may be more easily captured in the image. For instance, the granularity or structure of the feces sample can be better captured, resulting in improved performance and training of the machine learning model.

Optionally, the substrate is a sheet of paper, such as white paper, e.g. having a TAPPI brightness of 92 or higher. The sheet of paper can have a weight of about 50 - 200 g/m², such as about 80 g/m². The sheet of paper can have a surface roughness of about 80-300 mL/min measured using the Bendtsen method (ISO 8791-2) (e.g. using a Lorentzen & Wettre Roughness tester, applying constant compressed air (98 kPa), as specified in the SCAN-P 21 TAPPI UM 535 standard test method).

Optionally, the substrate may be integrated with fiducial marks, e.g. machine readable marks such as barcodes, like DataMatrix, QR code, UpCode, Nexcode, Aztec, or the like, along its margins to facilitate the accurate orientation and analysis of the fecal sample image. These fiducial marks or barcodes serve as reference points, enabling the imaging system to automatically adjust for rotation, skewing, scaling, and ensure consistent lighting and color conditions during image capture. The incorporation of such elements can significantly enhance the precision of image analysis, allowing for more reliable and reproducible results in the assessment of fecal samples. Furthermore, while white paper with specific properties, such as a TAPPI brightness of 92 or higher and a weight of about 50 - 200 g/m2, is commonly used, alternative materials like non-reflective matte synthetic sheets or coated fabrics can also be employed. These materials should ideally have a low gloss finish to minimize glare and ensure uniform light distribution, thereby aiding in maintaining the fidelity of the image captured for subsequent machine learning model training and analysis.

A sheet of paper is readily available and can provide easily sufficient contrast in the image of the sample of feces of the subject, under normal lighting condition. Additionally, by using a sheet of paper, a cost-effective solution can be obtained.

Optionally, the substrate and/or an area covered by the fecal streak have dimensions falling within a predetermined range.

In some examples, a sheet of paper with a predetermined size is used, for instance a sheet of paper in A4 format (or any other format). It is also possible that some region of a substrate is used for smearing the sample of feces. In some examples, a substrate is provided with a pre-marked region in which the feces sample has to be smeared. For example, a rectangle, circle, square, etc. region may be marked on a sheet of A4 paper. Optionally, the substrate may be printable by means of a printer based on an electronic format file, such as a Portable Document Format (PDF) file. This allows users for easily obtaining the substrate. Such electronic file may be easily provided to the user via a mobile app, such as an Android or iOS app or the like.

Optionally, the image classification is associated to abundances of predetermined metabolites.

Visual properties of the fecal samples, which can be identified by image detection models such as a trained machine learning model, can be linked to a subject's metabolite profile. It will be appreciated that in the macroscopic digital image an optical resolution per pixel can be larger than a dimension of individual metabolites. Hence, in the macroscopic digital image individual metabolites cannot be discerned. Nevertheless, it has been found that data indicative of one or more properties of the metabolites of the subject can be determined from the visual properties of the fecal samples in the macroscopic digital image.

The composition of the metabolomic profile in individuals is intricately linked to their overall health status, including the presence or risk of developing metabolic and other health-related conditions. Nutritional intake plays a pivotal role in this context, serving as a critical element in strategies aimed at monitoring and enhancing individual health, as well as in the prevention and management of various diseases, including metabolic disorders.

Enhanced health monitoring can be facilitated through the innovative use of fecal smear imagery on a suitable substrate, enabling the scalable assessment of health indicators across a broad population base. Individuals can conveniently contribute to this health monitoring effort by capturing and submitting images of their fecal samples through a mobile application.

These images can then be instantaneously analyzed via cloud-based servers employing advanced machine learning algorithms designed to interpret the metabolomic data. The resultant analytical outputs from the machine learning model may then undergo further refinement and interpretation, ultimately being communicated back to the individual and/or shared with healthcare professionals for informed clinical assessment and decision-making.

This integrated approach not only broadens the scope of health monitoring but also enhances the potential for early detection and intervention in health-related issues, thereby contributing to improved health outcomes. The composition of the metabolome in subjects may be associated to the health condition of the subject. The dietary composition can be a significant target in a strategy aiming at monitoring the health of the subject, or even prevention of metabolic diseases. The health monitoring can be made more easy by utilizing images of fecal streaks on a substrate. In this way, the scale of health monitoring can be improved, since a large number of subjects may provide their own images, for instance via a mobile application. The analysis can be performed on-the-fly using a mobile app. The images of the users may be uploaded to a server which is configured to analyze the images using the trained machine learning model. The classification output of the trained machine learning model may optionally be further post-processed and provided back to the user and/or to one or more health professionals.

Recent studies have observed a high variability in the effects on glucose tolerance to food between subjects although the meals or more specific food items were identical. For example, based on the analysis of the image of the fecal sample, a glucose response (as well as many other clinical parameters related to the health status) can be predicted by collecting data regarding personal features and/or a metabolite profile. Hence, a personalized diet (or a certain clinical intervention e.g. using a drug) may be easily built taking into account the analysis of the metabolite profile of the subject. In a similar fashion a personalized diet/ or intervention may be built for an animal.

Optionally, the estimated metabolites encompass a variety of digestion products resultant from the activities of distinct microbial enterotypes or abundance profiles.

Notably, genera such as Prevotella and Bacteroides play significant roles. The metabolic byproducts of these microorganisms are critical in assessing human nutritional status. For instance, Prevotella is known for producing short-chain fatty acids (SCFAs) like butyrate, which is essential for gut health and epithelial integrity; acetate, implicated in lipid metabolism and energy generation; and propionate, involved in gluconeogenesis and lipid management.

Additionally, Ruminococcus species contribute to fiber breakdown, generating SCFAs through the fermentation process, which are pivotal in maintaining gut health and metabolic functions. These microbes also produce other polysaccharide degradation products, contributing to the complex carbohydrate metabolism.

Lactobacillus and Bifidobacterium, recognized for fermenting carbohydrates, yield lactic acid, a fundamental metabolite in this process. These bacteria are also sources of B-vitamins, vital for numerous metabolic pathways, and bioactive peptides with potential immune-modulating properties.

Conversely, Clostridium perfringens, although a part of the normal gut flora, can produce harmful substances like alpha-toxin, besides its role in fermenting fibers to produce SCFAs in lesser quantities. Enterococcus species contribute to the metabolite pool by producing lactic acid and bacteriocins, which possess antimicrobial properties, playing a part in maintaining microbial balance and inhibiting pathogen overgrowth. Campylobacter species, often associated with protein metabolism, produce nitrogenous waste and potentially harmful toxins.

Expanding this, it is crucial to recognize the dynamic interplay between these microbes and their metabolic outputs, as they can significantly influence host health, affecting processes like immune regulation, nutrient absorption, and even the predisposition to certain diseases. For example, the balance between beneficial SCFA producers and potential pathogens like Clostridium perfringens and Campylobacter is vital for maintaining gut health and preventing dysbiosis, which can lead to conditions such as inflammatory bowel disease, obesity, and metabolic syndrome.

Optionally, the method includes determining, based on the output image classification, relative abundances of the one or more predetermined metabolites, such as but not limited to a ratio of one or more of the predetermined metabolites versus one or more others of the predetermined metabolites.

Optionally, the method includes assessing, based on the output from image classification, the diversity and composition metabolites in the subject's feces. Here, the term ' metabolite composition' refers to the array of metabolites produced by the gut microbiota and their relative abundances. A rich and balanced metabolite composition may indicate healthy digestive processes and a resilient gut environment.

Metabolite composition can provide insights into the metabolic functions occurring within the gut microbiome. For instance, a varied and balanced composition of short-chain fatty acids (SCFAs), amino acids, and other fermentation products suggests efficient nutrient processing and a symbiotic microbial community. Conversely, a predominance of certain metabolites or a lack of diversity in the metabolite profile might signal dysbiosis or an imbalance in gut microbial activities, which can be linked to health issues such as inflammatory bowel disease, metabolic disorders, or reduced immune function

Therefore, analyzing the metabolite composition in fecal samples can serve as an indicator of the metabolic health of the gut microbiome, reflecting the host's overall well-being and nutritional status. This analysis can also help in identifying specific metabolic pathways that are active or disrupted, offering potential targets for therapeutic intervention to restore or maintain gut health.

Optionally, the method encompasses devising a personalized nutrition regimen using the analytical data that reflects the metabolomic landscape within the subject's intestinal domain. Such a personalized approach to nutrition may pivot on the production and distribution of specific metabolites, rather than the microbial entities per se. For instance, the nutritional strategy could be tailored based on the abundance and ratios of key metabolites like short-chain fatty acids (SCFAs), indicative of a fiber-rich diet, or the presence of metabolic by-products associated with protein fermentation, which might signal a diet high in animal protein.

The personalized nutrition plan might leverage the quantification of metabolites such as butyrate, acetate, and propionate-products of carbohydrate fermentation by beneficial microbes-to enhance gut health and systemic wellness. Conversely, it could aim to mitigate the impact of metabolites associated with pathogenic bacteria, such as certain toxins or inflammatory markers, to address or preempt gastrointestinal disorders and other metabolic conditions.

Furthermore, the nutrition plan may seek to modify the metabolite profile, aiming to elevate the levels of beneficial metabolites like SCFAs, while reducing the prevalence of detrimental metabolites, thereby creating a metabolomic environment that promotes optimal health. The goal would be to shift the metabolite spectrum towards a profile that supports a balanced and healthful gut ecosystem, reflective of a nutrient-rich, fiber-dominant dietary intake.

In essence, by assessing the metabolic footprint within the gut, this method enables the identification of various patterns and health status, providing a foundation upon which personalized medical and/or nutritional interventions can be structured. The ultimate aim of such personalized care would be to recalibrate the metabolite composition towards a more favorable balance, promoting overall health and mitigating the risk of various diseases.

Optionally, the method encompasses devising a personalized health plan to improve a subject's overall physical and mental well-being.

Optionally, the method for analyzing the metabolite profile of the subject is repeated over time. Such repeated analysis provides information on the effectivity of any changes in the determined data indicative of the one or more properties of the metabolite profile of the subject, such as metabolite abundance profiles, as a result of any changes to the subject's nutrition.

A bi-directional relationship between the metabolite profile and the subject can be vital to health. The disclosure enables easy and efficient monitoring data indicative of the composition of the metabolome. An enhanced understanding of such relationship can be used for improving the health of the subject. Additionally or alternatively, it can be employed for better understanding disease, which can result in improved therapeutic approaches.

Optionally, the acquisition of the digital image of the fecal sample may be performed under white light illumination, offering a user-friendly means of image capture while yielding reliable insights into the intestinal metabolite profile. White light illumination ensures uniform lighting across the entire visible spectrum, facilitating a consistent and accurate representation of the sample.

Alternatively, the digital image may be obtained using narrow band illumination, which can be tailored to accentuate specific attributes of the metabolome. This illumination strategy may involve either the selective attenuation of certain wavelength ranges within white light or the employment of specific narrow wavelength bands, thereby enhancing the discriminatory power for particular metabolomic features.

In another optional embodiment, the digital image captures the autofluorescence inherent in the fecal sample. This autofluorescence is typically elicited by exposing the sample to light sources that excite fluorescent emissions, such as those in the blue and/or ultraviolet spectrum. Images capturing this autofluorescence can offer augmented information, providing a more nuanced perspective on the metabolic characteristics and compositional nuances of the metabolite profile.

Optionally, the machine learning model has been trained using a data set comprising a plurality of digital images of fecal samples, each digital image accompanied by metabolite measurement data representative of abundances of the predetermined metabolites in the fecal sample in the digital image.

Optionally the data set includes digital images obtained under white light illumination, narrow band illumination or autofluorescence excitation illumination. In one embodiment, the machine learning model has been trained using a data set comprising a plurality of digital images obtained under white light illumination accompanied by the metabolite measurement data, for processing user digital images obtained under white light illumination. In another embodiment, the machine learning model has been trained using a data set comprising a plurality of digital images obtained under narrow band illumination accompanied by the metabolite measurement data, for processing user digital images obtained under narrow band illumination. In another embodiment, the machine learning model has been trained using a data set comprising a plurality of digital images obtained under autofluorescence excitation illumination accompanied by the metabolite measurement data, for processing user digital images obtained under autofluorescence excitation illumination.

Optionally, the measurement data includes gas chromatography-mass spectrometry data, corresponding to the fecal sample in the digital image.

Optionally, the measurement data accompanying each digital image is statistically normalized to a predetermined total abundance of the predetermined metabolites.

Optionally, the data set used for training is augmented using one or more image transformations, such as cropping, rotation, translation, and color change.

According to an aspect, the disclosure provides for a method of determining an indication of a subject's health based on data indicative of one or more properties of the metabolome of the subject determined according to any of the preceding claims.

Advantageously, the method extends its utility beyond dietary interventions to encompass a broader spectrum of medical applications, addressing a range of disorders including cardiometabolic, neurological, and other systemic conditions. For example, the consumption patterns of saturated fats and animal proteins, prevalent in Western diets, have correlations not only with metabolic disorders like obesity, type 2 diabetes, and cardiovascular diseases but also with alterations in the metabolite profile, such as diminished metabolite diversity. Moreover, the method's applicability in crafting personalized dietary plans can be instrumental in mitigating cardiometabolic risks, demonstrating its preventive and therapeutic potential.

Furthermore, the method's adaptability allows for the exploration of interventions in neurological disorders, where gut-brain axis interactions play a crucial role, indicating the influence of metabolite profiles on neurological health and disease progression. This holistic approach enables the method to be applied in diverse medical interventions, tailoring dietary and therapeutic strategies to individual metabolic and physiological profiles, thereby optimizing health outcomes across a wide array of medical conditions.

Optionally, the digital image of the sample of feces of the subject is taken by means of a mobile device such as a mobile phone with camera and uploaded to a server, wherein the server is configured to carry out the method according to the disclosure.

In this way, a user friendly method can be obtained, reducing the barrier for (frequent) monitoring of the metabolite profile. The users themselves may provide the images, for instance by uploading the images via a mobile app. In some cases, this would not require any additional tools. For example, the substrate may be a sheet of paper, which is available to users. Furthermore, the camera of the mobile device may be used for capturing the digital image of the fecal sample. The tools used for getting the fecal sample (e.g. scoop) or smearing the fecal sample on the substrate for obtaining a fecal streak (e.g. blade) may also be easily available to the users. In some examples, these tools may be provided to the users by means of a kit of parts. This user friendly method of the user providing the digital image using a mobile device optionally uses a trained machine learning model that relies on an algorithm in which the varying circumstances of this relatively random user acquisition of the digital image versus a prescribed and/or professional, i.e. trained, acquisition of the digital image have been taken into account as much as possible. For example, the algorithm used for the trained machine learning model may optionally have parameters compensating at least to some degree for lighting condition varieties in the provided digital images, image quality variations, or user errors in sample preparation, Alternatively, or additionally, the method may involve prescribing to the user a desired or necessary way of obtaining and providing the digital image. For example, a mobile app may be used to this end. This can optionally be the same mobile app as the mobile app for uploading the images. The mobile app can for example contain and show exemplary images as guidance to the user for providing a useable digital image for the method. The mobile app may also provide a suitable template configured to assist the user in providing the digital image in a necessary or desired orientation, crop size, image size, contrast, and/or other image properties. Alternatively, or additionally, a physical template may be provided to the user, for example as part of a kit of parts, to assist the user in providing the digital image in a correct manner. The template may have fiducial marks or barcodes along its margins to facilitate the accurate orientation and analysis of the digital image. These fiducial marks or barcodes serve as reference points, enabling the imaging system to automatically adjust for rotation, scaling, and ensure consistent lighting and color conditions during image capture. Alternatively, or additionally, software may be used for image processing, such as software configured to enhance an image quality of a digital image taken by the user. The software may optionally enable calibration of digital images adapted to enable a desired image classification. The software may be provided to the user for use in the method at the user end, for example by means of a mobile app. Alternatively, or additionally, the software may reside at a server end and be configured to automatically enhance an image quality of an uploaded digital image.

Optionally, the method involves a step of providing feedback to the user of the data indicative of one or more properties of the metabolite profile of the subject based on the output image classification. The feedback may for example comprise a notification to the user of the determined data indicative of one or more properties of the metabolite profile of the subject. The notification may include information with respect to a health profile of the subject. The notification may include dietary information suited to the subject. The notification may in particular include dietary suggestions to the subject.

Optionally, the method involves combining the data indicative of one or more properties of the metabolite profile of the subject based on the output image classification together with other health monitoring data. In particular, the method may involve combining the data indicative of one or more properties of the metabolite profile of the subject based on the output image classification together with data of the intestinal microflora of the subject. The combination of data indicative of one or more properties of the metabolite profile of the subject together with data of the intestinal microflora of the subject provides for an efficient and accurate determination of the subject's digestive functioning, enabling a convenient way of improving on the subject's dietary needs for a healthy life.

Optionally, the method is used for monitoring temporal metabolite profile variations. The machine learning model, for instance a deep neural network model, may be used to track changes in fecal metabolites in relation to fecal images over time.

In one embodiment, this disclosure presents a methodology for training a machine learning model to analyze the intestinal metabolite profile of a subject using digital images of fecal samples. The process entails:
a) Acquiring a dataset comprising numerous digital images of fecal samples;
b) Collecting measurement data indicative of the concentrations of specific metabolic profiles and/or clinical metadata related to the health status of the subject (e.g. human or animal). Additional measurement information may also include microbial or fungal composition measurements, related omics-data, enterotypes, species, present in the fecal samples or samples derived from any other tissue (including but not limited to saliva, skin, blood, urine, biopsy) from the same subject as in each digital image;
c) Utilizing the data gathered in step b) alongside the digital images from step a) to train the machine learning model, thereby equipping it to autonomously correlate metabolic profiles and/or clinical metadata, omics measurements, and additional structured as well as unstructured information with digital images of fecal samples.

For optimal results, the digital images employed in training the machine learning model should be macroscopic, with a recommended field of view ranging from at least 30 mm by 30 mm to upwards of 150 mm by 150 mm. These images are ideally captured using high-resolution cameras, including those found in mobile devices like smartphones, to ensure detailed and accurate representations of the fecal samples.

Traditional methods for determining the composition of the human gut microbiota and its metabolomic profile, such as gas chromatography-mass spectrometry or DNA/RNA sequencing, are resource-intensive. Leveraging digital imaging of fecal matter, particularly fecal streaks, can significantly enhance the efficiency of training machine learning models. This approach not only reduces the complexity and resources required but also offers a cost-effective alternative for the ongoing monitoring and analysis of the metabolite profiles in individuals, thus providing a scalable and accessible solution for health monitoring and personalized medical interventions.

According to an aspect, the disclosure provides for a system for analyzing a metabolite profile of a subject, the system comprising: one or more processors for receiving a digital image of a sample of feces of the subject; and a memory storing a trained machine learning model, wherein the trained machine learning model is configured to output a classification based on the digital image and/or one or more features extracted from the digital image provided as input; and wherein the one or more processors are configured to determine, data indicative of one or more properties of the metabolite profile of the subject based on the output image classification.

Preferably, the digital image is a macroscopic image. The macroscopic image can e.g. have a field of view of at least 30 mm wide and at least 30 mm tall. The digital image can e.g. have a field of view of at least 75 mm wide and at least 75 mm tall, such as at least 150 mm wide and at least 150 mm tall. The digital image can advantageously be obtained using a camera of a mobile device, such as a mobile phone.

The system offers a streamlined and efficient method for identifying key metabolites that play a pivotal role in long-term metabolic health. In one aspect, the disclosure pertains to a computer program product that, when executed on a computing device, facilitates the implementation of the disclosed method. This program product is designed to streamline the process of analyzing the metabolome, providing a user-friendly interface for data processing and interpretation.

Furthermore, the disclosure encompasses a trained machine learning model, specifically tailored for analyzing metabolome of a subject based on macroscopic images. This model is adept at processing macroscopic fecal sample images, utilizing them to generate classifications that reflect various attributes of the metabolite profile. Notably, the machine learning model employs an algorithm capable of classifying images beyond its initial training set, thereby enhancing its predictive and analytical capabilities. This advanced feature allows the model to extend its learning from a limited dataset, traditionally acquired through expert analysis and laboratory methods, to a broader array of digital images.

The architecture of the trained machine learning model may include multiple layers, employing specific rules and algorithms to enhance its analytical precision. For example, it might utilize convolutional layers for image processing, recurrent neural networks for pattern recognition in sequential data, and deep learning algorithms to autonomously identify complex patterns and correlations within the data. The configuration of the model could be optimized to facilitate accurate classification, pattern recognition, and predictive analysis, thus enabling a comprehensive and detailed assessment of the metabolite profile for personalized health insights and interventions.

By covering a broad spectrum of machine learning techniques and rchitectures, the model provide a robust and versatile tool for the accurate analysis of intestinal health and metabolic functions, paving the way for targeted dietary and therapeutic strategies based on subjects (for example human or animal) metabolomic profiles.

Advantageously, the system can provide for a rapid and simple mechanism for identifying metabolites which are important in long-term metabolic health.

According to an aspect, the disclosure provides for a computer program product configured for performing, when run on a computer, the steps of the method according to the disclosure.

According to an aspect, the disclosure provides for a trained machine learning model, the trained machine learning model being configured for use in a method for analyzing the metabolome of a subject, wherein the trained machine learning model is configured to receive an image of a fecal sample of a subject as input and provide an image classification as output, the image classification being indicative of one or more properties of the metabolite profile of the subject. The trained machine learning model may rely on an algorithm configured to classify images not directly trained on. The algorithm may enable the machine learning model trained with only a practical number of data sets obtained from expert analysis, e.g. using lab techniques, to be trained further with digital images.

Optionally, the computer program is a mobile app which is executable on a mobile device, such as a mobile phone or a tablet. In some examples, the computer program is executable on a personal computer, such as a Windows, Macintosh (Apple), Linux, and/or Unix machine.

According to an aspect, the disclosure provides for a processor implemented method for determining a metabolite profile of a subject, comprising: receiving, by one or more processors, a digital image of a sample of feces of the subject; and determining, by the one or more processors, the metabolite profile of the subject on the basis of inferred patterns between the image and its corresponding metabolite profile obtained for example by gas chromatography-mass spectrometry procedure. Optionally, the pattern determination is performed using a machine learning data processing model that has been trained to associate abundances of the predetermined metabolites with digital images of fecal samples.

According to an aspect, the disclosure provides for a system for determining a metabolite profile of a subject, comprising: one or more processors for receiving a digital image of a sample of feces of the subject; a memory storing a machine learning data processing model that has been trained to associate abundances of predetermined metabolites with digital images of fecal samples; wherein the one or more processors are configured to determine, on the basis of the digital image and using the machine learning data processing model, the metabolite profile of the subject.

A deep learning neural network operates as a method of representation learning, characterized by multiple layers of representation, derived through the composition of simple, yet nonlinear modules. These modules sequentially transform the data from its raw form into increasingly abstract representations at successive levels. This architecture enables the network to discern complex patterns that may be elusive to traditional analytical methods. Consequently, instead of developing a code for the specific task of identifying visual patterns in fecal sample images, the neural network can be trained to adaptively process a diverse and evolving array of fecal imagery, utilizing classification algorithms. By using training data, the deep neural network can establish an effective classification framework, thereby enhancing its capability to analyze the metabolite composition and metabolite diversity of the subject efficiently.

It will be appreciated that any of the aspects, features and options described in view of the methods apply equally to the systems and the described devices, computer program products. It will also be clear that any one or more of the above aspects, features and options can be combined.

### BRIEF DESCRIPTION OF THE DRAWING

The disclosure will further be elucidated on the basis of exemplary embodiments which are represented in a drawing. The exemplary embodiments are given by way of non-limitative illustration. It is noted that the figures are only schematic representations of embodiments of the disclosure that are given by way of non-limiting example.

In the drawing:
Fig. 1 shows a schematic diagram of a system;
Fig. 2 shows a schematic diagram of a system;
Fig. 3 shows a schematic diagram of a substrate with a fecal streak applied thereon;
Fig. 4 shows a schematic diagram of a substrate;
Fig. 5a, 5b show an image of a fecal streak on a substrate;
Fig. 6 shows a schematic diagram of a method;
Fig. 7a shows an image of a sample of feces on a substrate obtained under narrow band illumination; and
Fig.7b shows an image of autofluorescence of a sample of feces on a substrate.

### DETAILED DESCRIPTION

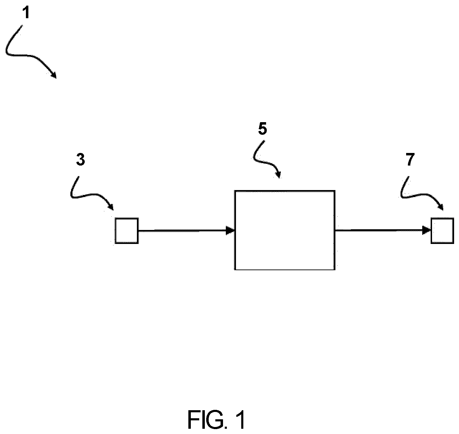

Fig. 1 shows a schematic diagram of a system 1 for analyzing a metabolite profile of a subject. The system 1 comprises one or more processors for receiving a digital image 3 and/or one or more features extracted from the digital image 3, which is provided as input to trained machine learning model 5, wherein the trained machine learning model 5 is configured to output a classification 7 based on the digital image 3 and/or one or more features extracted from the digital image 3. The one or more processors are configured to determine, data indicative of one or more properties (e.g. abundance levels) of the metabolite profile of the subject based on the output image classification 7.

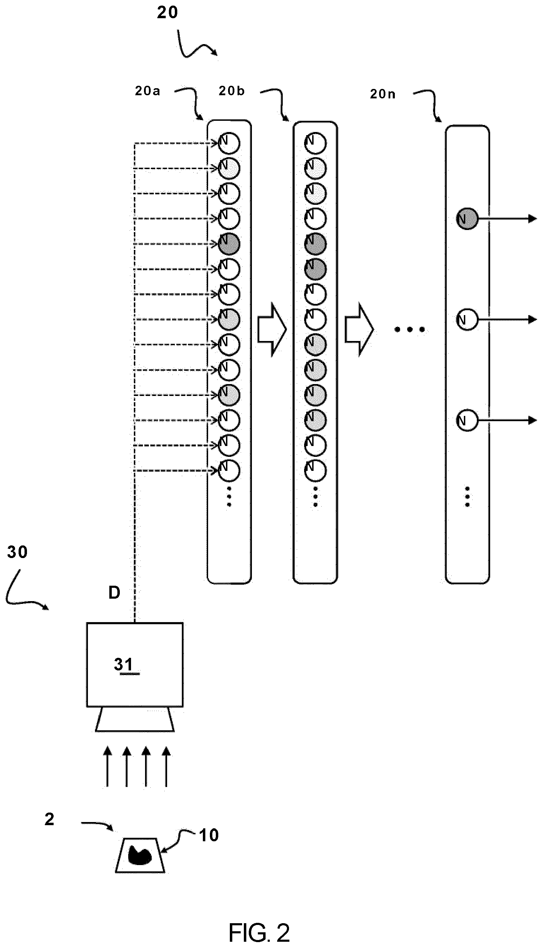

Figure 2 illustrates a schematic representation of a deep neural network 20, consisting of numerous neurons "N" arranged across several layers 20a-n, creating a structured deep architecture. This network typically comprises an input layer 20a, an output layer 20n, and multiple intermediate hidden layers 20b-(n-1). The neural network, once trained, is adept at computing the output values Ox, Oy, Oz for the neurons in the output layer 20n, based on a sequence of input values derived from the fecal sample image 2 of the subject. In this depicted scenario, the fecal sample 2 rests on a surface 10, with its image captured by a sensor unit 30, which may be a digital visual camera or a similar device. While this camera can be an independent unit, it's feasible to utilize the camera of a mobile device, such as a smartphone or tablet. The image capture typically occurs under normal lighting conditions, positioning the camera 31 perpendicularly to the surface holding the fecal sample 2, employing white light illumination. However, to enhance image quality, particularly when using mobile devices, a corrective transformation of the captured image is beneficial to offset visual distortions caused by the angle of the camera relative to the surface. This adjustment is crucial, especially when the alignment between the camera and the surface is less than ideal, thereby improving the accuracy of subsequent image analyses.

In the shown example, the neural network 20 comprises an input layer 20a with neurons configured to receive input values corresponding to one or more images of the sample of the feces of the subject on a surface (e.g. substrate). For instance, the feces can be put on a substrate, such as for instance a piece of paper. In some examples, the sample of feces is smeared out on the substrate/surface such as to become a streak of feces. It will be appreciated that other sensory data or data derived therefrom can be used as input. In some embodiments, features extracted from the images (e.g. by performing image processing steps) are provided as input to the deep neural network.

In some embodiments, e.g. as shown, interconnections between the neurons "N" are formed exclusively across subsequent layers, e.g. between the input layer 20a and the first hidden layer 20b, between the first hidden layer 20b and the second hidden layer 20c (not shown), etc., up to the last hidden layer (index n-1, not shown) and the output layer 20n. Also other configurations of networks and connections may be envisaged.

A controller of the system may be configured to communicate with the sensor unit 30 comprising at least one sensor device, e.g. camera 31, for registering sensor data representing a sample of the feces of the subject. In some examples, the camera 31 is placed above the sample of feces and a top image is taken. The controller can be also configured to comprise or communicate with the neural network 20. For example, neural network 20 is configured (and/or programmed) to receive the sensor data D, and process the sensor data D to calculate the one or more output values Ox, Oy, Oz, etc. (e.g. classification values representing a respective classification of the sample of feces).

Many other types sensor devices may be envisaged. Also combinations of two or more sensors can be used as input to a neural network (e.g. deep neural network). The two or more sensors may be of the same type or different types. For example, the system may comprise a number of different cameras, e.g. combination of visual camera and infrared camera.

Based on the sensor data registered by the sensor unit 30, the neural network may classify the data. In some embodiments, the classifications may be predetermined, and the neural network may be trained to recognized the classifications (supervised learning). In other embodiments, the neural network may itself determine a set of classifications that may then be labelled (unsupervised learning). In some examples, the output of the neural network may calculate a probability of classifications indicative of or related to one or more properties of the metabolite profile of the subject.

The classification values Ox, Oy, Oz may serve as input to a further system or module which is to advise certain actions, such as for example health monitoring, personalized diet proposals, etc. It may also be served as input to further system components (not shown) which may take action (e.g. alert a health professional) or present options based on the classification. The classification values Ox, Oy, Oz may also be simply output to a user interface reporting the event (not shown).

Aspects of the present disclosure may also relate to corresponding methods of training a neural network. In one embodiment, the method comprises classifying one or more properties of the metabolite profile of the subject based on an image of the sample of feces of the subject. In some examples, a (non-transitory) computer-readable medium can be provided with software instructions that, when executed, causes performing the method as described herein, or forming a network or computer system as described herein. It will be appreciated that analyzing steps may also be performed on cloud systems or platforms (e.g. server).

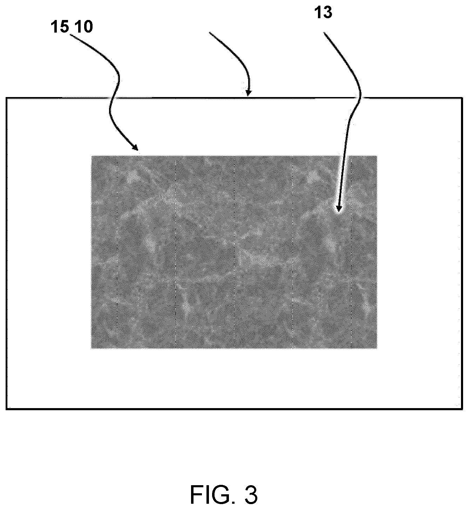

Fig. 3 shows a schematic diagram of a substrate 10 with a fecal streak 13 applied thereon. The fecal streak 13 has been applied on a portion or region 15 of the substrate 10. The substrate 10 with fecal streak 13 can be effectively used for analyzing and estimating the metabolites of a subject. A digital image of the substrate 10 with fecal streak 13 can be taken. The digital image and/or one or more features extracted from the digital image may be provided as input to a trained machine learning model which is configured to output a classification based on said input digital image and/or one or more features extracted from the digital image. Data indicative of one or more properties of the metabolome of the subject can be determined based on the output image classification.

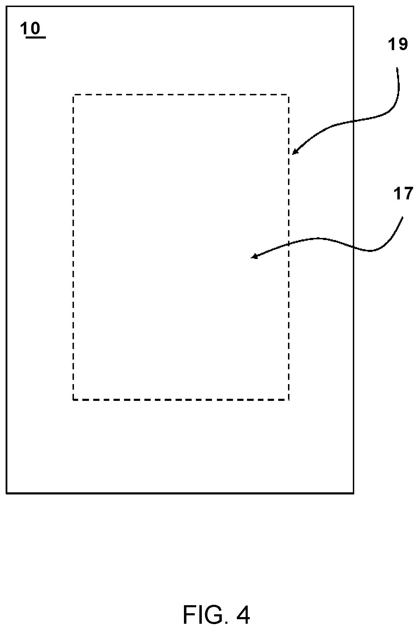

Fig. 4 shows a schematic diagram of a substrate 10. The substrate 10 has a region 17 which is visually indicated by marking lines 19 (dashed line) in which the fecal streak is to be provided. The substrate 10 may be a sheet of paper (e.g. standard A4 format), allowing the user to easily produce it (e.g. by printing a digital document such as a PDF document, Word Document, or the like). This is particularly advantageous when fecal samples are frequently monitored. The user can perform the analysis by means of a mobile device with an app. The app can be configured to receive the image of the sample of feces and upload it to a server or the cloud for analysis by means of the trained machine learning model. As in this example the sheet of paper is of A4 format, the field of view of the digital image is approximately 30 x 21 cm. Hence, in this example, the digital image is a macroscopic image. Other dimensions of the field of view are possible. The macroscopic digital image can e.g. have a field of view of at least 30 mm wide and at least 30 mm tall. The macroscopic digital image can e.g. have a field of view of at least 75 mm wide and at least 75 mm tall, such as at least 150 mm wide and at least 150 mm tall. The macroscopic digital image can advantageously be obtained using a camera of a mobile device, such as a mobile phone. The mobile phone camera can e.g. have 48 megapixels, i.e. about 8000x6000 pixels.

One or more results from the analysis can be fed back to the user. This also enables to track the development of the state of the metabolome of the subject over time (e.g. periodically sampling, such as for instance with time intervals of days, weeks or months).

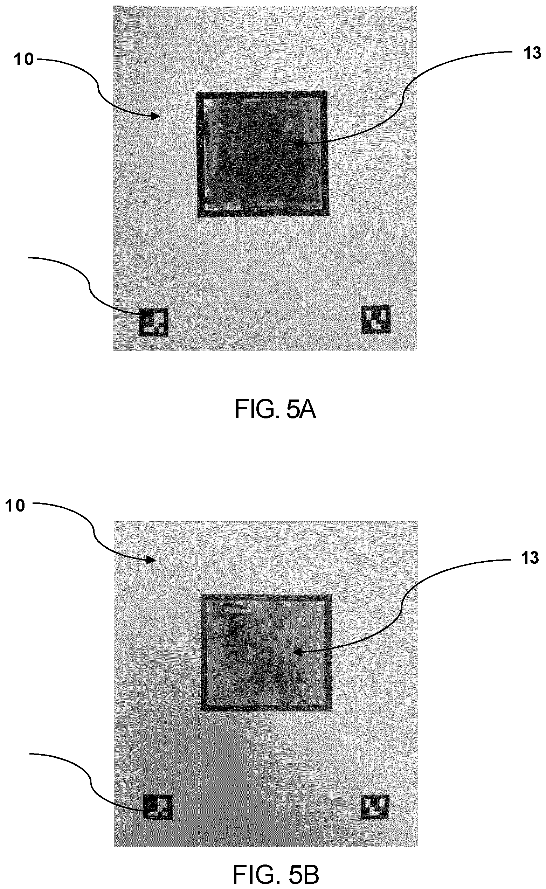

Figures 5a and 5b display images of fecal streaks 13 on a substrate 10, illustrating variations in metabolite profiles derived from different enterotypes. The substrate 10 contains fiducial marks 16 along its margins, here in the bottom two corners of the substrate template, to facilitate the accurate orientation and analysis of the digital image. Instead, 1, 3, 4 or more fiducial marks may be used, for example 4 marks in each corner of the substrate template. These fiducial marks or barcodes serve as reference points, enabling the imaging system to automatically adjust for rotation, scaling, and ensure consistent lighting and color conditions during image capture.

In Figure 5a, the fecal streak is characterized by metabolites predominantly produced by the Prevotella enterotype, whereas Figure 5b shows metabolites typically associated with the Bacteroides enterotype. These distinctions in metabolite composition may reflect underlying health conditions, with temporal changes in metabolite profiles potentially serving as biomarkers for health status.

The analysis of these fecal streak images utilizes deep neural network models to discern subtle shifts in fecal metabolite compositions over time, linking these changes to the overall gut metabolite landscape. Comparative evaluations using fecal samples have been conducted, aligning fecal imaging with traditional analytical techniques such as mass spectrometry and gas chromatography. The machine learning model, informed by these comparisons, achieved significant predictive accuracy, with an AUC above 0.82, indicating a strong correlation between fecal imaging and the metabolite composition of the gut microbiota.

This approach demonstrates that deep learning models can detect patterns and correlations with specific metabolites, including short-chain fatty acids, which are typically undetectable to the naked eye. The digital images used in this research are macroscopic, captured on A4-sized substrates, where the metabolites themselves are not directly visible. Instead, it is the broader compositional and textural features of the fecal matter that enhance the machine learning model's ability to classify the metabolite profiles accurately.

The substrate, such as A4 paper in these examples, ensures that the analysis remains consistent and reliable under various imaging conditions, including different backgrounds and lighting scenarios, thereby affirming the method's effectiveness in identifying and categorizing the nuanced features indicative of specific metabolite profiles in the samples.

In the example of Fig. 5a, 5b, the image classification is associated particularly to metabolite abundance profiles. This proves useful in assessing quality of human and animal nutrition. It is also possible that the image classification is associated to metabolite diversity. Metabolite diversity refers to the number of different metabolites in the feces and how evenly they are represented. A high metabolite diversity, i.e. a high number of different metabolites that are evenly spread across the gut metabolome, is representative of a healthy, resilient gut.

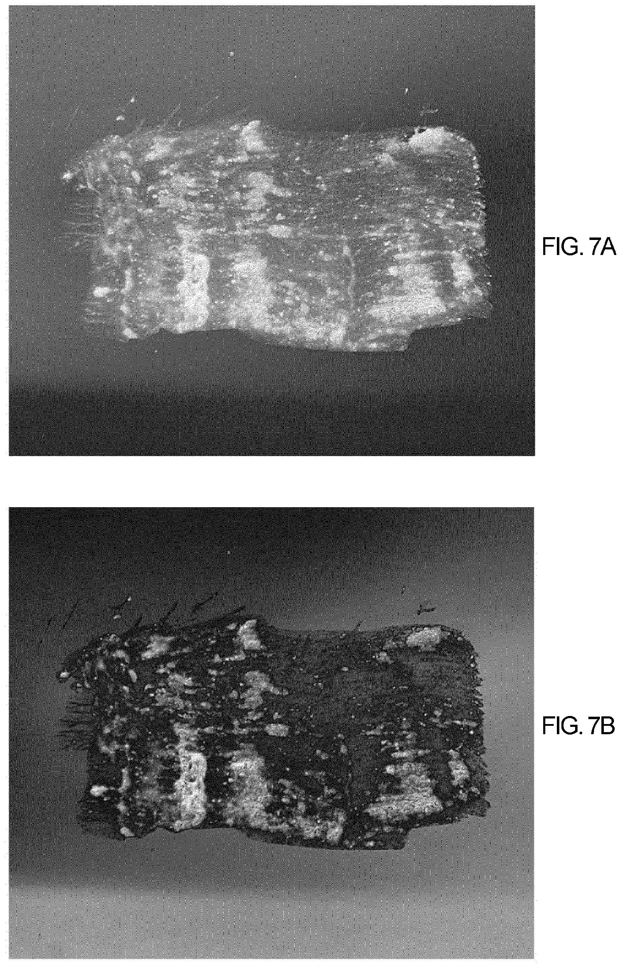

In the example of Fig. 5a, 5b, the digital images are obtained under white light illumination. However, different illumination may be used. In the example of Fig. 7a, the digital image of the sample of feces is obtained under narrow band illumination. The narrow band illumination can be used to more effectively discriminate certain properties of the metabolites. In the example of Fig.7b the digital image includes autofluorescence of the sample of feces. The autofluorescence is in this example obtained by illuminating the sample of feces with light exciting the autofluorescence, here in the ultraviolet range. It has been found that the digital image including autofluorescence can provide enhanced data indicative of one or more properties of the metabolite profile.

The machine learning model can be trained on chemical or molecular data and digital images of the fecal streaks (example above) samples, such as to identify metabolite properties such as fecal structure as well as presence and relative abundance of metabolite types, or ratios between certain types in the sample. Advantageously, the machine learning model is used for visual evaluations of stool samples.

In an example, a dataset of fecal images is collected and subsequently used for training an image detection machine learning model. The dataset may contain images of fecal samples spread out on substrate, e.g. on a standard white A4 paper. The illumination of the images in the dataset may e.g. be white light illumination, narrow band illumination, and/or autofluorescence excitation illumination. Particularly, a separate dataset of white light illumination, narrow band illumination, or autofluorescence excitation illumination images may be provided for training the image detection machine learning model for use with white light illumination, narrow band illumination, or autofluorescence excitation illumination images, respectively. The images may be accompanied by chemical or molecular data. The machine learning model can identify visual properties of the fecal samples which are linked to a subject's metabolite profile and/or subject's health. Such information can be subsequently used to establish nutritional guidelines based on images of the feces only. Hence, personalized nutrition can be determined based on the determined data indicative of the one or more properties of the metabolome of the subject. Advantageously, a highly efficient and effective system can be obtained in this way.

In some examples, the training is performed with focus on the short fatty acids to toxins ratio. Alternatively, or additionally, training may be performed with focus on metabolite diversity.

In some examples, during training the images are augmented , i.e., transformed using random transformations, e.g. cropping, rotations, translations, color changes, or the like. The advantage of this is the models are able to learn more general properties of the images, instead of small subtle differences in form of noise. It improves the model's ability to generalize, or in other words be able to perform well on unseen data. It is also possible to obtain one or more sub-images from the macroscopic digital image which sub-images may be used efficiently for further learning. For example the macroscopic image of feces of a subject may be divided into segments, for instance using a predetermined template, with each segment representing a sub-image of the macroscopic digital image. Optionally the sub-images can be analyzed for usability or not for the training of the system and/or for use in the methods such as input for the learning model.

In some examples, the chemical and/or molecular data may be preprocessed. For example the gas chromatography and/or mass spectrometry data may be normalized to a point where each sample has equal read count. In some examples, all ASV's with less than 0.1 * N non-zero read counts were dropped, where N is the sample size. In some examples, all ASV's with an average read count of less than 3 were dropped. However, other values may also be used for these exemplary optional steps.

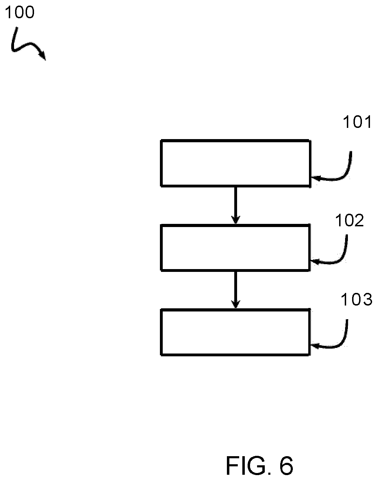

Fig. 6 shows a schematic diagram of a method 100 for analyzing the metabolite profile of a subject. In a first step 101, a digital image of a sample of feces of the subject is received by one or more processors. In a second step, the digital image and/or one or more features extracted from the digital image is provided as input to a trained machine learning model which is configured to output a classification based on said input digital image and/or one or more features extracted from the digital image. In a third step 103, data indicative of one or more properties of the metabolite profile of the subject is determined based on the output image classification.

In some examples, a mobile device app is used, wherein the mobile device app is configured to allow uploading of a digital image of the sample of feces, preferably spread out on white paper. The images can be analyzed in real-time by the system according to the disclosure (e.g. cloud). In some examples, the users may automatically receive nutritional guidelines and a reasonable estimate of the overall health status of their gut metabolome based on the visual analysis performed by means of the trained machine learning model. However, it is also envisaged that the method is carried out by means of a website or software executed on a computer. Various implementations are possible.

It will be appreciated that various machine learning models may be used. In some examples, a deep learning model is employed. Deep learning allows computational models that are composed of multiple processing layers to learn representations of data with multiple levels of abstraction. In this way, visual pattern recognition may be significantly improved. Deep learning discovers intricate structure in large data sets by using the backpropagation algorithm to indicate how a machine should change its internal parameters that are used to compute the representation in each layer from the representation in the previous layer. Various deep learning networks and architectures may be employed.

It will be appreciated that the subject can be a human or animal (e.g. cattle). For both humans and animals an improved personalized diet can be more easily obtained, taking into account the condition of the intestinal metabolome of the subject. The individual health condition of the subjects can thus be significantly improved in an easy way by employing the method according to the disclosure.

It will be appreciated that the term metabolome may be understood as all metabolites found in the feces of the subject.

In some examples, the digital image is directly provided as input to the trained machine learning model. Optionally, the digital image is first preprocessed prior to providing it as input to the trained machine learning model, for example involving segmentation (selection of certain portions of data in the digital image overlapping with one or more identified features in the image; filtering; etc). In some example, the pixels of the digital image are provided to the nodes of the input layer of the trained deep learning model.

It will be appreciated that the method may include computer implemented steps. All above mentioned steps can be computer implemented steps. Embodiments may comprise computer apparatus, wherein processes performed in computer apparatus. The disclosure also extends to computer programs, particularly computer programs on or in a carrier, adapted for putting the disclosure into practice. The program may be in the form of source or object code or in any other form suitable for use in the implementation of the processes according to the disclosure. The carrier may be any entity or device capable of carrying the program. For example, the carrier may comprise a storage medium, such as a ROM, for example a semiconductor ROM or hard disk. Further, the carrier may be a transmissible carrier such as an electrical or optical signal which may be conveyed via electrical or optical cable or by radio or other means, e.g. via the internet or cloud.

Some embodiments may be implemented, for example, using a machine or tangible computer-readable medium or article which may store an instruction or a set of instructions that, if executed by a machine, may cause the machine to perform a method and/or operations in accordance with the embodiments.

It will be further understood that when a particular step of a method is referred to as subsequent to another step, it can directly follow said other step or one or more intermediate steps may be carried out before carrying out the particular step, unless specified otherwise. Likewise it will be understood that when a connection between components such as neurons of the neural network is described, this connection may be established directly or through intermediate components such as other neurons or logical operations, unless specified otherwise or excluded by the context.

Various embodiments may be implemented using hardware elements, software elements, or a combination of both. Examples of hardware elements may include processors, microprocessors, circuits, application specific integrated circuits (ASIC), programmable logic devices (PLD), digital signal processors (DSP), field programmable gate array (FPGA), logic gates, registers, semiconductor device, microchips, chip sets, et cetera. Examples of software may include software components, programs, applications, computer programs, application programs, system programs, machine programs, operating system software, mobile apps, middleware, firmware, software modules, routines, subroutines, functions, computer implemented methods, procedures, software interfaces, application program interfaces (API), methods, instruction sets, computing code, computer code, et cetera.

The graphics and/or image/video processing techniques may be implemented in various hardware architectures. Graphics functionality may be integrated within a chipset. Alternatively, a discrete graphics processor may be used. For example, processing of images (still or video) may be performed by a graphics subsystem such as a graphics processing unit (GPU) or a visual processing unit (VPU). As still another embodiment, the graphics or image/video processing functions may be implemented by a general purpose processor, including e.g. a multi-core processor. In a further embodiment, the functions may be implemented in a consumer electronics device. Embodiments, using a combination of different hardware architectures are possible.

In various embodiments, the controller can communicate using wireless systems, wired systems, or a combination of both. When implemented as a wired system, the system may include components and interfaces suitable for communicating or wired communications media, such as input/output (I/O) adapters, physical connectors to connect the I/O adapter with a corresponding wired communications medium. When implemented as a wireless system, the system may include components and interfaces suitable for communicating over a wireless shared media, such as one or more antennas, transmitters, receivers, transceivers, amplifiers, filters, control logic, and so forth. An example of wireless shared media may include portions of a wireless spectrum, such as the RF spectrum and so forth. A wireless communication device may be included in order to transmit and receive signals using various suitable wireless communications techniques. Such techniques may involve communications across one or more wireless networks. Exemplary wireless networks include, but are not limited to, cellular networks, wireless local area networks (WLANs, cfr. WiFi), wireless personal area networks (WPANs), wireless metropolitan area network (WMANs), satellite networks, et cetera. In communicating across such networks, the transmitter may operate in accordance with one or more applicable standards in any version.

Herein, the disclosure is described with reference to specific examples of embodiments of the disclosure. It will, however, be evident that various modifications, variations, alternatives and changes may be made therein, without departing from the essence of the disclosure. For the purpose of clarity and a concise description features are described herein as part of the same or separate embodiments, however, alternative embodiments having combinations of all or some of the features described in these separate embodiments are also envisaged and understood to fall within the framework of the disclosure as outlined by the claims. The specifications, figures and examples are, accordingly, to be regarded in an illustrative sense rather than in a restrictive sense. The disclosure is intended to embrace all alternatives, modifications and variations which fall within the scope of the appended claims. Further, many of the elements that are described are functional entities that may be implemented as discrete or distributed components or in conjunction with other components, in any suitable combination and location.

In the claims, any reference signs placed between parentheses shall not be construed as limiting the claim. The word 'comprising' does not exclude the presence of other features or steps than those listed in a claim. Furthermore, the words 'a' and 'an' shall not be construed as limited to 'only one', but instead are used to mean `at least one', and do not exclude a plurality. The mere fact that certain measures are recited in mutually different claims does not indicate that a combination of these measures cannot be used to an advantage.

## Claims

1. A method for analyzing a metabolite profile of a subject, comprising:
receiving, by one or more processors, a macroscopic digital image of a fecal streak of a sample of feces of the subject on a substrate;
providing the macroscopic digital image and/or one or more features extracted from the macroscopic digital image as input to a trained machine learning model which is configured to output an image classification based on said input macroscopic digital image and/or one or more features extracted from the macroscopic digital image; and
determining, by the one or more processors, data indicative of one or more properties of the metabolite profile of the subject based on the output image classification.

2. The method of claim 1, wherein the substrate contains fiducial markers or barcodes configured to enhance image analysis accuracy and consistency.

3. The method of claim 1 or claim 2, wherein the substrate's material properties, such as color, texture, and absorbency, are standardized to optimize image analysis, and preferably is a sheet of paper.

4. The method of any one claims 1-3, wherein the macroscopic digital image has a field of view of at least 30 x 30 mm, preferably at least 75 x 75 mm, more preferably at least 150 x 150 mm.

5. The method of any of claims 1-4, wherein the image classification is associated to abundances of predetermined metabolites, optionally including identifying and quantifying metabolite biomarkers associated with specific dietary patterns or health conditions.

6. The method of any of claims 1-5, including comparing the determined data indicative of one or more properties of the metabolite profile of the subject with established healthy benchmarks to identify potential nutritional deficiencies or excesses, optionally including determining a personalized nutrition based on the identification.

7. The method of any of claims 1-6, wherein the digital image of the sample of feces is obtained under white light illumination, or under narrow band illumination, or under autofluorescence excitation illumination.

8. The method of any of claims 1-7, wherein the machine learning model incorporates temporal data analysis to track changes in metabolite profiles over time, aiding in longitudinal health and dietary studies.

9. The method of any of claims 1-8, wherein the machine learning model has been trained using a data set comprising a plurality of digital images of fecal samples, each digital image accompanied by metabolite profile data representative of abundances of the predetermined metabolites in the fecal sample in the digital image.

10. The method of claim 9, wherein the data set includes gas chromatography-mass spectrometry data, corresponding to the fecal sample in the digital image.

11. The method of claim 9 or 10, wherein the data accompanying each digital image is statistically normalized to a predetermined total abundance of the predetermined metabolite.

12. A method of determining an indication of a subject's health based on data indicative of one or more properties of the metabolite profile of the subject determined according to any of the preceding claims 1-11, optionally integrating the data with other health indicators to formulate a holistic health overview.

13. The method according to claim 12, wherein the digital image of the sample of feces of the subject is taken by means of a mobile device and uploaded to a server, wherein the server is configured to carry out the method according to any one of the claims 1-11.

14. A method for training a machine learning model for analyzing a metabolite profile of a subject with digital images of fecal samples, the method including:
a) receiving a data set comprising a plurality of digital images of fecal samples;
b) receiving data representative of abundances of the predetermined metabolites in the fecal sample in each digital image of the plurality of digital images; and
c) training the machine learning data processing model based on the date received in step b) and the digital images received in step a) for enabling, after completion of the training period, the step of automatically associating abundances of predetermined metabolites with digital images of fecal samples.

15. A system for analyzing a metabolite profile of a subject, the system comprising:
one or more processors for receiving a macroscopic digital image of a sample of feces of the subject; and
a memory storing a trained machine learning model, wherein the trained machine learning model is configured to output an image classification based on the macroscopic digital image and/or one or more features extracted from the macroscopic digital image provided as input; and
wherein the one or more processors are configured to determine, data indicative of one or more properties of the metabolite profile of the subject based on the output image classification.
